(19) Europäisches Patentamt
European Patent Office
Office européen des brevets

(11) Veröffentlichungsnummer : **0 451 104 A2**

(12) **EUROPÄISCHE PATENTANMELDUNG**

(21) Anmeldenummer : **91810218.7**

(51) Int. Cl.$^5$ : **C08G 65/40**, C08G 61/12

(22) Anmeldetag : **26.03.91**

(30) Priorität : **03.04.90 CH 1105/90**
**10.01.91 CH 45/91**

(43) Veröffentlichungstag der Anmeldung :
**09.10.91 Patentblatt 91/41**

(84) Benannte Vertragsstaaten :
**DE ES FR GB IT NL SE**

(71) Anmelder : **CIBA-GEIGY AG**
**Klybeckstrasse 141**
**CH-4002 Basel (CH)**

(72) Erfinder : **Pfaendner, Rudolf, Dr.**
**Sackgasse 3**
**W-6149 Rimbach/Odenwald 1 (DE)**
Erfinder : **Kainmüller, Thomas, Dr.**
**Oetlingerstrasse 2**
**CH-4057 Basel (CH)**
Erfinder : **Hoffmann, Kurt, Dr.**
**Heidenbergstrasse 15**
**W-6147 Lautertal 2 (DE)**
Erfinder : **Wolf, Jean-Pierre, Dr.**
**257, chemin de la motta**
**CH-1791 Courtaman (CH)**
Erfinder : **Kramer, Andreas, Dr.**
**Bundtels**
**CH-3186 Düdingen (CH)**
Erfinder : **Stockinger, Friedrich**
**Au Fernotz**
**CH-1784 Courtepin (CH)**

(54) **Lösliche Polyarylenetherketone.**

(57) In halogenierten Kohlenwasserstoffen lösliche Polyarylenetherketone mit einer reduzierten Viskosität von 0,1 bis 2,0 dl/g, gemessen bei 25°C an einer 1-%igen Lösung in N-Methylpyrrolidon (NMP), die, bezogen auf die Gesamtmenge der im Polyarylenetherketonharz vorhandenen Strukturelemente, 1-100 Mol-% eines wiederkehrenden Strukturelementes der Formel I oder II

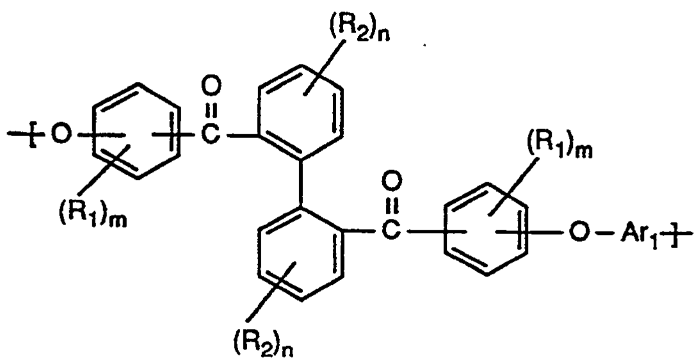

(I),

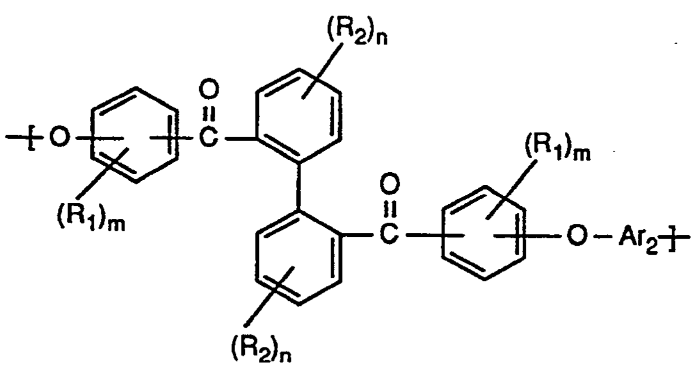

(II)

und 99-0 Mol-% eines wiederkehrenden Strukturelementes der Formel III

EP 0 451 104 A2

$$-\!\!\left[O-Ar_2-O-Ar_1\right]\!\!- \quad \text{(III)},$$

enthalten, worin $R_1$ ein Alkyl, Alkoxy, Aryl, Alkenyl oder Halogenatom bedeutet, wobei m für Null oder eine Zahl von 1-4 steht, $R_2$ ein $C_1$-$C_4$-Alkyl, $C_1$-$C_4$-Alkoxy, $C_6$-$C_{12}$-Aryl oder Halogenatom bedeutet, wobei n für Null oder eine Zahl von 1-4 steht, $Ar_1$ ein zweiwertiger Rest einer diphenolischen Verbindung und $Ar_2$ ein zweiwertiger Rest einer aktivierten, zur nucleophilen Austauschreaktion befähigten Dihalogenverbindung sind, können vorteilhaft aus der Lösung heraus verarbeitet werden und eignen sich zum Modifizieren von Matrixharzen.

Die vorliegende Erfindung betrifft in halogenierten Kohlenwasserstoffen lösliche Polyarylenetherketone enthaltend 2,2′-Bis-(benzoyl)-biphenyl-Einheiten, Verfahren zu deren Herstellung, die aus den erfindungsgemässen Polyarylenetherketonen hergestellten Formstoffe, Beschichtungen oder Folien sowie die Verwendung der erfindungsgemässen Polyarylenetherketone zum Modifizieren von Matrixharzen.

Polyarylenetherketone sind technische Werkstoffe mit sehr guten mechanischen und thermischen Eigenschaften, die in der Regel in halogenierten Kohlenwasserstoffen unlöslich sind oder instabile Lösungen bilden. Beispielsweise sind Polyarylenetherketone mit 4,4′-Bis-(benzoyl)-biphenyl-Einheiten aus der EP-A-0 194 062 bekannt. Diese ketogruppenhaltigen Polyether weisen eine besonders gute Lösungsmittelbeständigkeit, beispielsweise gegenüber Methylenchlorid, auf.

Für manche Anwendungen, insbesondere bei der Modifizierung von duromeren Matrixharzen, sind Polyetherketone erforderlich, die verbesserte Löslichkeitseigenschaften aufweisen, ohne dass die thermischen Eigenschaften, wie Wärmeformbeständigkeit, d.h., die Glasübergangstemperatur, erheblich reduziert werden. Es wurde nun gefunden, dass Polyarylenetherketone mit 2,2′-Bis-(benzoyl)-biphenyl-Einheiten überraschend in gebräuchlichen organischen Lösungsmitteln, vorzugsweise in halogenierten Kohlenwasserstoffen, gut löslich sind und stabile Lösungen bilden.

Gegenstand der vorliegenden Erfindung sind somit in halogenierten Kohlenwasserstoffen lösliche Polyarylenetherketone mit einer reduzierten Viskosität von 0,1 bis 2,0 dl/g, gemessen bei 25°C an einer 1-%igen Lösung in N-Methylpyrrolidon (NMP), die, bezogen auf die Gesamtmenge der im Polyarylenetherketonharz vorhandenen Strukturelemente, 1-100 Mol-% eines wiederkehrenden Strukturelementes der Formel I oder II

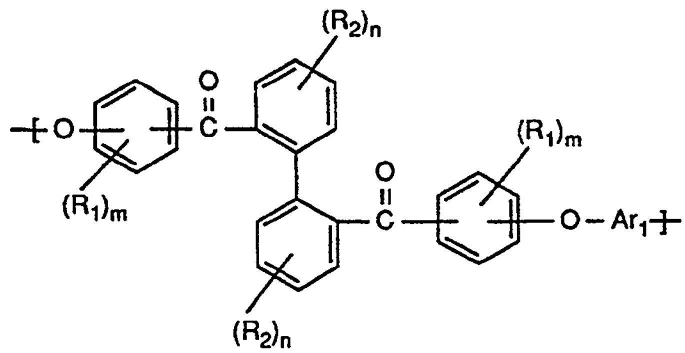

(I),

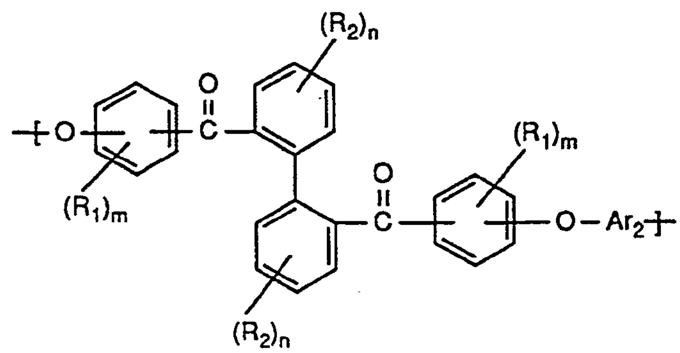

(II)

und 99-0 Mol-% eines wiederkehrenden Strukturelementes der Formel III

$$\left[ O\text{-}Ar_2\text{-}O\text{-}Ar_1 \right] \quad (III)$$

enthalten, worin $R_1$ ein $C_1$-$C_4$-Alkyl, $C_1$-$C_4$-Alkoxy, $C_6$-$C_{12}$-Aryl, $C_2$-$C_4$-Alkenyl oder Halogenatom bedeutet, wobei m für Null oder eine Zahl von 1-4 steht, $R_2$ ein $C_1$-$C_4$-Alkyl, $C_1$-$C_4$-Alkoxy, $C_6$-$C_{12}$-Aryl oder Halogenatom bedeutet, wobei n für Null oder eine Zahl von 1-4 steht, $Ar_1$ ein zweiwertiger Rest einer diphenolischen Verbindung und $Ar_2$ ein zweiwertiger Rest einer aktivierten, zur nucleophilen Austauschreaktion befähigten Dihalogenverbindung sind.

Der Rest $Ar_1$ in den Strukturelementen der Formeln I und III steht vorzugsweise für eine unsubstituierte oder durch ein oder mehrere $C_1$-$C_4$-Alkyle, $C_1$-$C_4$-Alkoxy oder Halogenatome substituierte Gruppe der Formeln IVa bis IVg

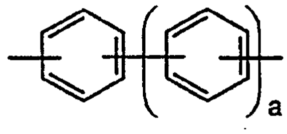 (IVa),

wobei a Null oder die Zahl 1 ist,

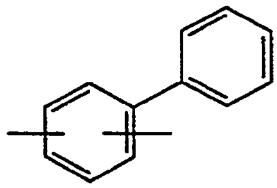 (IVb), 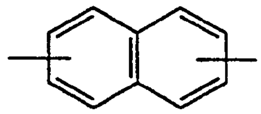 (IVc),

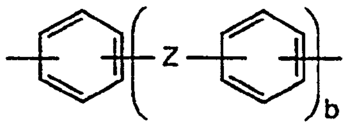 (IVd),

wobei b die Zahl 1 oder 2 ist,

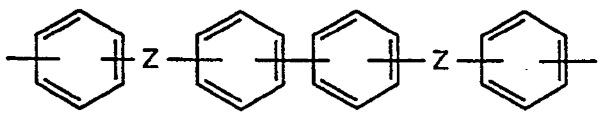 (IVe),

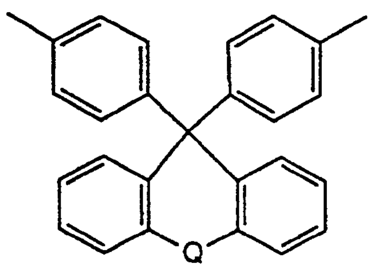 (IVf) oder

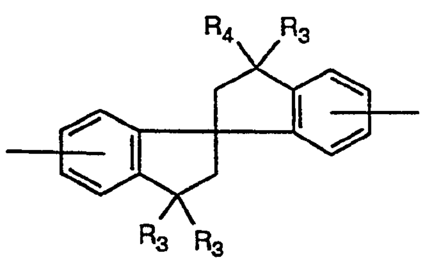 (IVg)

darstellt, worin Z für -CO-, $-SO_2-$, -SO-, -S-, -O-, $-\overset{|}{C}(CH_3)_2$ , $-\overset{|}{C}(CF_3)_2$ , $-CH_2-$, $-\underset{C_6H_5}{\overset{|}{\underset{|}{C}}}-CH_3$ oder $-\underset{R_4}{\overset{|}{\underset{|}{P}}}O$ , Q für eine direkte Bindung, -O-, $-CH_2-$ oder -CO-, $R_3$ je für ein

$C_1$-$C_4$-Alkyl und $R_4$ für ein $C_1$-$C_4$-Alkyl oder Phenyl stehen.

Insbesondere stellt $Ar_1$ eine unsubstituierte oder durch ein oder mehrere $C_1$-$C_4$-Alkyle, $C_1$-$C_4$-Alkoxy oder Halogenatome substituierte Gruppe der Formeln IVa bis IVe

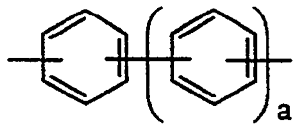 (IVa),

wobei a Null oder die Zahl 1 ist,

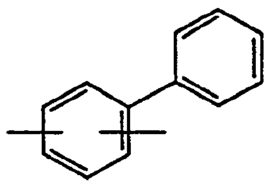 (IVb), 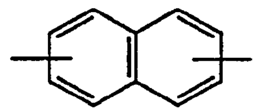 (IVc),

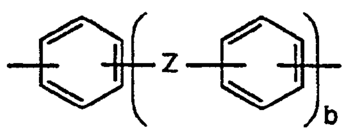 (IVd),

wobei b die Zahl 1 oder 2 ist, oder

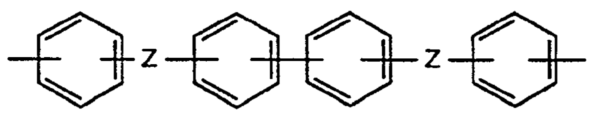 (IVe)

dar, worin Z für -CO-, $-SO_2-$, -SO-, -S-, -O-, $-C(CH_3)_2-$, $-C(CF_3)_2-$, $-CH_2-$, oder $-C(CH_3)(C_6H_5)-$ steht.

Der Rest $Ar_2$ in den Strukturelementen der Formeln II und III steht vorzugsweise für eine unsubstituierte oder durch ein oder mehrere $C_1$-$C_4$-Alkyle, $C_1$-$C_4$-Alkoxy oder Halogenatome substituierte Gruppe der Formel IVh bis IVl

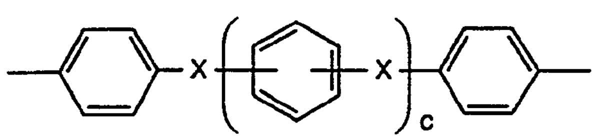 (IVh),

wobei c für Null oder die Zahl 1 oder steht,

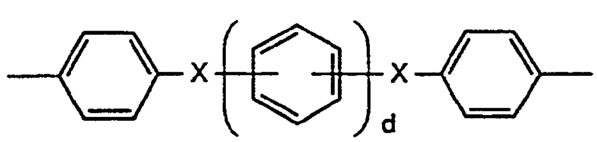 (IVi),

wobei d für die Zahl 2 oder 3 und X für -CO-, $-SO_2-$ oder -SO- stehen,

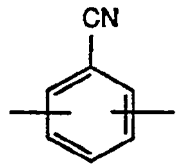

(IVj),

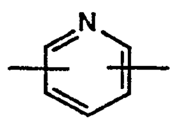

(IVk) oder

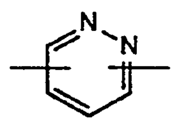

(IVl).

Insbesondere stellt $Ar_2$ eine unsubstituierte oder durch ein oder mehrere $C_1$-$C_4$-Alkyle, $C_1$-$C_4$-Alkoxy oder Halogenatome substituierte Gruppe der Formel IVh oder IVi

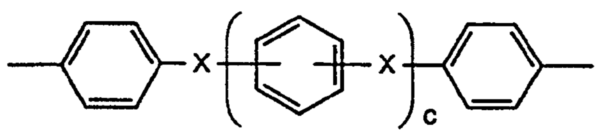

(IVh),

wobei c für Null oder die Zahl 1 oder steht,

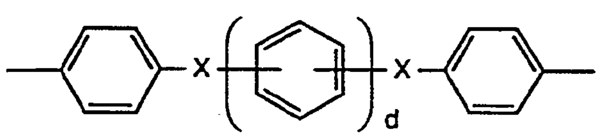

(IVi),

wobei d für die Zahl 2 oder 3 und X für -CO-, $-SO_2-$ oder -SO- stehen.

Vorzugsweise enthalten die erfindungsgemässen Polyarylenetherketone 5-100 Mol-% eines wiederkehrenden Strukturelementes der Formel I oder II und 95-0 Mol-% eines wiederkehrenden Strukturelementes der Formel III.

Insbesondere sind in den erfindungsgemässen Polyarylenetherketonen 10-100 Mol-% eines wiederkehrenden Strukturelementes der Formel I oder II und 90-0 Mol-% eines wiederkehrenden Strukturelementes der Formel III enthalten. In den erfindungsgemässen Polyarylenetherketonen sind die Strukturelemente der Formeln I, II und III vorzugsweise unsubstituiert.

Beispiele für bevorzugte Reste $Ar_1$ in den Strukturelementen der Formel I und III sind

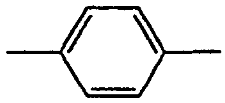 , 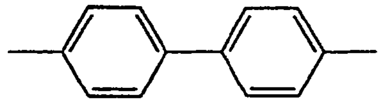 , 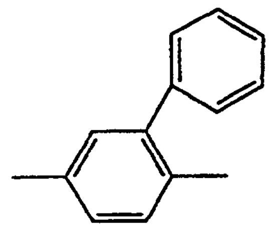 ,

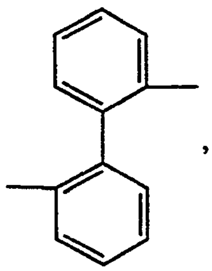 , 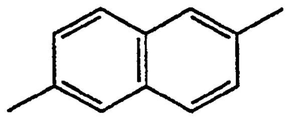 , 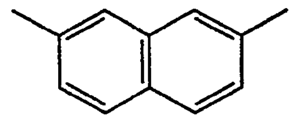 ,

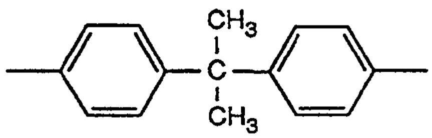 , 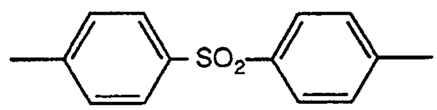 ,

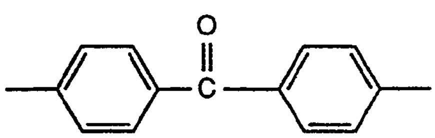 , 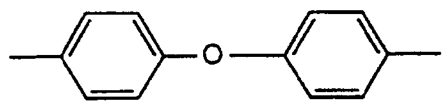 ,

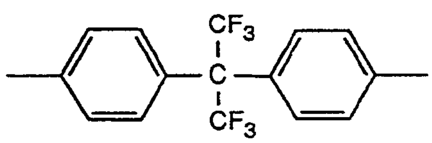 , 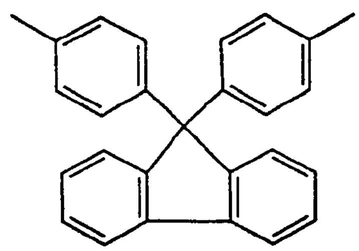 oder

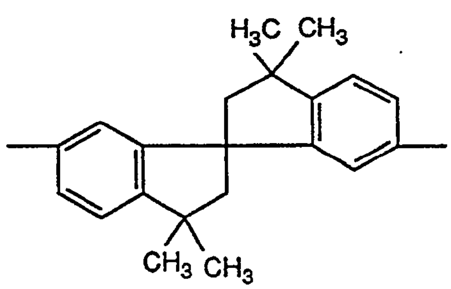 .

Ganz besonders bevorzugte Reste $Ar_1$ sind solche der Formeln

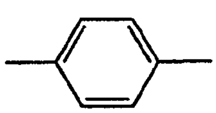 , 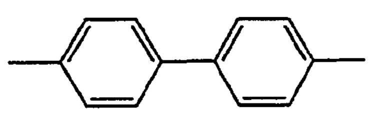 , 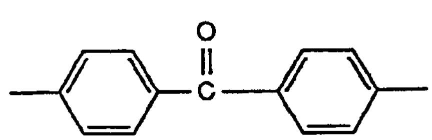 ,

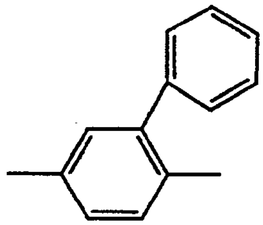 oder 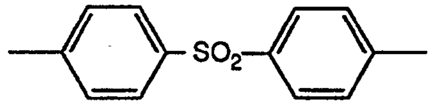 .

Beispiele für bevorzugte Reste $Ar_2$ in den Strukturelementen der Formel II und III sind

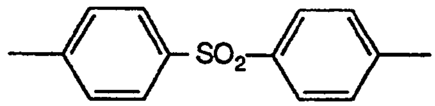 , 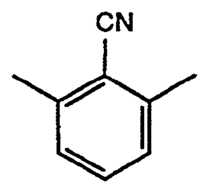 oder

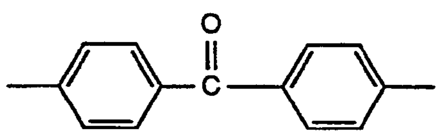 .

Die erfindungsgemässen Polyarylenetherketone können hergestellt werden, indem man

(a) eine Dihalogenverbindung der Formel V

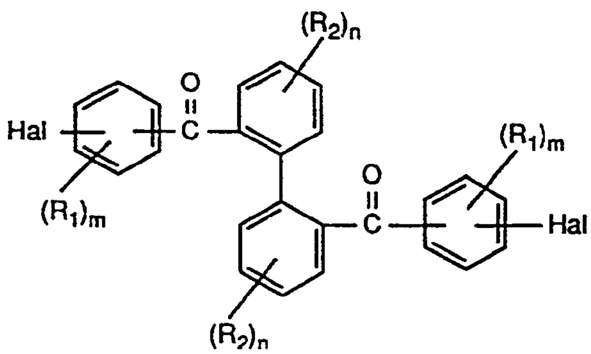 (V),

worin $R_1$, $R_2$, m und n die gleiche Bedeutung wie in Formel I haben und Hal für ein Halogen, insbesondere Fluor oder Chlor, steht, oder ein Gemisch aus einer Dihalogenverbindung der Formel V und einer darin bis zu 99 Mol-% enthaltenden Dihalogenverbindung der Formel VI

$$\text{Hal-Ar}_2\text{-Hal} \qquad \text{(VI)},$$

worin $Ar_2$ die gleiche Bedeutung wie in Formel II hat und Hal für ein Halogen, vorzugsweise Fluor oder Chlor, steht, in äquimolaren Mengen mit einem Diphenol der Formel VII oder dessen Alkali- oder Erdalkaliphenolat

$$\text{HO-Ar}_1\text{-OH} \qquad \text{(VII)},$$

worin $Ar_1$ die gleiche Bedeutung wie in Formel I hat, in Gegenwart von Alkali und in einem aprotischen Lösungsmittel polykondensiert oder

(b) eine Verbindung der Formel VIII oder deren Alkali- oder Erdalkaliphenolate

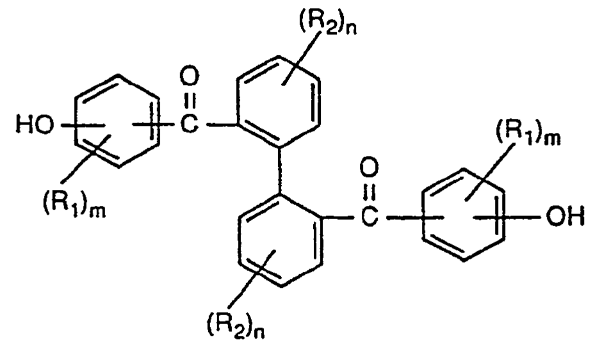

(VIII),

worin $R_1$, $R_2$, m und n die gleiche Bedeutung wie in Anspruch 1 haben, oder ein Gemisch aus einer Verbindung der Formel VIII und einem darin bis zu 99 Mol-% enthaltenen Diphenol der Formel VII in äquimolaren Mengen mit einer Dihalogenverbindung der Formel VI in Gegenwart von Alkali und in einem aprotischen Lösungsmittel polykondensiert, bis das erhaltene Polyarylenetherketon eine reduzierte Viskosität von 0,1 bis 2,0 dl/g aufweist, gemessen an einer 1-%igen Lösung in NMP (1 g Polymeres in 100 ml NMP) bei 25°C.

Vorzugsweise polykondensiert man (a) eine Dihalogenverbindung der Formel V oder ein Gemisch aus einer Dihalogenverbindung der Formel V und einer darin bis zu 95 Mol-%, insbesondere bis zu 90 Mol-%, enthaltenen Dihalogenverbindung der Formel VI in äquimolaren Mengen mit einem Diphenol der Formel VII oder (b) eine Verbindung der Formel VIII oder ein Gemisch aus einer Verbindung der Formel VIII und einem darin bis zu 95 Mol-%, insbesondere bis zu 90 Mol-% enthaltenen Diphenol der Formel VII in äquimolaren Mengen mit einer Dihalogenverbindung der Formel VI in Gegenwart von Alkali und in einem aprotischen Lösungsmittel polykondensiert, bis das erhaltene Polyarylenethersulfon eine reduzierte Viskosität von 0,15 bis 1,8 dl/g, insbesondere 0,2-1,5 dl/g, aufweist.

Unter äquimolaren Mengen versteht man im Zusammenhang mit dem oben angegebenen Verfahren ein Molverhältnis der Dihalogenverbindung der Formel V oder der Dihalogenverbindungen der Formeln V und VI zu dem Diphenol der Formel VII bzw. ein Molverhältnis der Verbindung der Formel VIII oder der Verbindungen der Formeln VII und VIII zu der Dihalogenverbindung der Formel VI von 0,9 bis 1,1. Bevorzugt ist ein Molverhältnis von 0,95 bis 1,05.

Als Alkali verwendet man in diesem Verfahren in der Regel Alkali- und Erdalkalicarbonate, wie Natrium-, Kalium- oder Calciumcarbonat; doch können auch andere alkalische Reagentien, wie Natriumhydroxid, Kaliumhydroxid oder Calciumhydroxid, Verwendung finden.

Polare, aprotische Lösungsmittel, die beim Verfahren zur Herstellung der erfindungsgemässen Polyetherharze eingesetzt werden können, sind beispielsweise Dimethylsulfoxid, Dimethylacetamid, Diethylacetamid, Tetramethylharnstoff, N-Methylcaprolactam, N-Methylpyrrolidon und bevorzugt Diphenylsulfon.

Die Reaktion wird zweckmässig bei erhöhter Temperatur durchgeführt, vorzugsweise bis zur Rückflusstemperatur des Lösungsmittels, also etwa bis 350°C.

Häufig empfiehlt sich die Mitverwendung eines Schleppmittels, wie z.B. Chlorbenzol, Xylol oder Toluol, um das bei der Umsetzung gebildete Wasser azeotrop aus dem Reaktionsgemisch entfernen zu können.

Die Verbindungen der Formeln V und VIII sind bekannt. Beispielsweise wird die Herstellung von 2,2'-Bis-(4-fluorbenzoyl)-biphenyl im Journal of American Chemical Society 1935, 57, Seite 1095 ff beschrieben, und die Herstellung von 4,4'-Dichlor-2,2'-bis-(o-chlorbenzoyl)-biphenyl wird im Journal of Organic Chemistry 1984, 49(2), 296-300, beschrieben. Die Herstellung von beispielsweise 2,2'-Bis-(4-hydroxybenzoyl)-biphenyl wird im Journal of Chemical Society, C, 1969, 2388 ff und im Journal of Chemical Society 1938, 1561 ff beschrieben. Die bekannten Herstellungsverfahren für die Verbindungen der Formeln V und VIII ergeben nur Ausbeuten von etwa 30 % der Theorie.

Es wurde ferner gefunden, dass man die Verbindungen der Formeln V und VIII in bedeutend höheren Ausbeuten erhält, wenn man bei der Herstellung der Biphenylverbindungen von einer 2-Jodbenzophenonverbindung ausgeht und diese in Gegenwart von Kupferpulver auf etwa 200°C erhitzt.

Gegenstand vorliegender Erfindung ist somit auch ein Verfahren zur Herstellung von Verbindungen der Formeln V und VIII, dadurch gekennzeichnet, dass man eine Verbindung der Formel IX

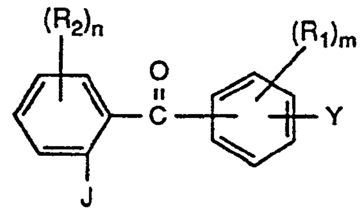

(IX),

worin $R_1$, $R_2$, m und n die gleiche Bedeutung wie in Formel V oder VIII haben und Y für ein Halogenatom oder $C_1$-$C_4$-Alkoxy, vorzugsweise Fluor, Chlor oder $C_1$-$C_2$-Alkoxy, insbesondere Fluor oder -$OCH_3$, steht in Gegenwart einer 0,5 bis 3-fachen Gewichtsmenge Kupferpulver, bezogen auf die Gewichtsmenge der Verbindung der Formel IX, auf 150-250°C erhitzt, wobei eine Verbindung der Formel X

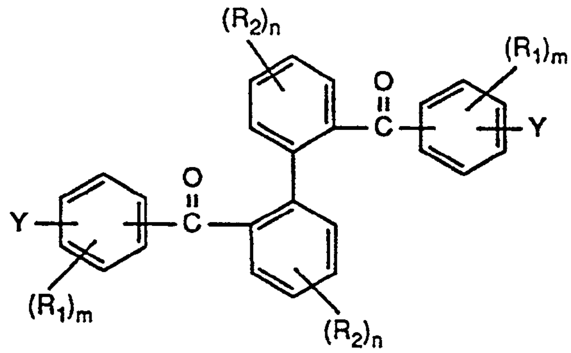

(X)

erhalten wird, worin $R_1$, $R_2$, m, n und Y die gleiche Bedeutung wie in Formel IX haben, und wenn Y für ein $C_1$-$C_4$-Alkoxy steht, diese Verbindung mit $AlCl_3$ in einem organischen Lösungsmittel unter Rühren bei etwa 60-160°C zu einer Verbindung der Formel VIII umsetzt, wobei pro 1 Mol der Verbindung der Formel X 3-6 Mole $AlCl_3$ eingesetzt werden.

Als organisches Lösungsmittel verwendet man dabei vorzugsweise Benzol, Toluol, Xylol oder Chlorbenzol.

Die Verbindungen der Formel IX können hergestellt werden, indem man eine Verbindung der Formel XI

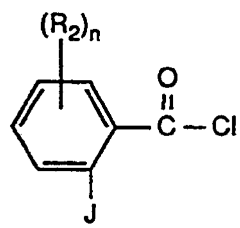

(XI),

worin $R_2$ und n die gleiche Bedeutung wie in Formel IX haben, beispielsweise 2-Jodbenzoylchlorid, in Gegenwart einer katalytischen Menge $FeCl_3$ mit einer Verbindung der Formel XII

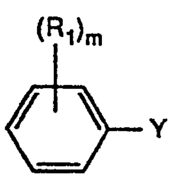

(XII),

worin $R_1$, m und Y die gleiche Bedeutung wie in Formel IX haben, beispielsweise Fluorbenzol oder Anisol, umsetzt.

Die Dihalogenverbindungen der Formel VI sind bekannt, beispielsweise aus der DE-OS 30 14 230 und der EP-A-0 001 879. Geeignete Dihalogenverbindungen der Formel VI sind beispielsweise 4,4'-Dichlordiphenylsulfon, 4,4'-Difluordiphenylsulfon, 1,4-Bis-(4-chlorphenylsulfonyl)-benzol, 4,4'-Bis-(4-chlorphenylsulfonyl)-biphenyl und 2,6-Bis-(4-chlorphenylsulfonyl)-naphthalin. Ferner werden 2,6- oder 2,7-Di-(p-fluorbenzoyl)-naphthalin und 2,6- oder 2,7-Di-(p-chlorbenzoyl)-naphthalin in der DE-OS 38 04 159 und in POLYMER, 1988, Band 29, Seite 358 ff beschrieben. Weitere geeignete Dihalogenverbindungen sind beispielsweise 2,6-Difluor- oder 2,6-Dichlorbenzonitril.

Die Diphenolverbindungen der Formel VII stellen ebenfalls bekannte Verbindungen dar und sind grösstenteils im Handel erhältlich. Beispiele für geeignete zweiwertige Phenole der Formel VII sind Hydrochinon, Resorcin, 4,4′-Dihydroxybiphenyl, 2,5-Dihydroxybiphenyl, 4,4′-Dihydroxydiphenylether, 4,4′-Dihydroxydiphenylsulfon, 4,4′-Dihydroxy-3,3′,5,5′-tetramethyldiphenylsulfon, 4,4′-Dihydroxybenzophenon, 4,4′-Dihydroxydiphenylmethan, 4,4′-Dihydroxydiphenylthioether, 2,2-Di-(4-hydroxyphenyl)-propan oder Dihydroxynaphthalin. Ferner sind 2,6-Bis-(4-hydroxybenzoyl)-naphthalin und 2,7-bis-(4-hydroxybenzoyl)-naphthalin aus der DE-OS 38 04 159 bekannt, und chlor- oder methylsubstituiertes 2,6-Bis-(4-hydroxybenzoyl)-naphthalin aus den US-Patenten 4 447 592 und 4 275 226. Weitere geeignete zweiwertige Phenole sind beispielsweise 9,9-Bis-(4-hydroxyphenyl)-fluoren und 6,6′-Dihydroxy-3,3,3′,3′-tetramethyl-1,1′-spirobiindan.

Wie eingangs erwähnt, sind die erfindungsgemässen Polyarylenetherketone in gebräuchlichen organischen Lösungsmitteln, vorzugsweise in halogenierten, d.h., chlorierten oder fluorierten, Kohlenwasserstoffen, insbesondere in chlorierten Kohlenwasserstoffen, wie beispielsweise Methylenchlorid, Trichlormethan, Dichlorethan, Trichlorethan oder Chlorbenzol löslich. Die erfindungsgemässen Polyarylenetherketone sind auch in polaren aprotischen Lösungsmitteln, wie N-Methylpyrrolidon, N,N-Dimethylformamid, Dimethylsulfoxid und Sulfolan, oder meistens auch in cyclischen Ethern, wie Tetrahydrofuran oder Dioxan, sowie auch in Cyclohexanon oder Cyclopentanon löslich. Aufgrund ihrer Löslichkeit können die erfindungsgemässen Polyarylenetherketone vorteilhaft aus einer Lösung heraus zu Filmen verarbeitet oder in andere Matrixsysteme eingebracht werden.

Die Lösungen der erfindungsgemässen Polymerverbindungen sind über mehrere Wochen stabil, das heisst, es erfolgt weder eine Trübung, ein Niederschlag oder die Ausfällung des Polymeren. Die Erfindung betrifft daher auch eine Lösung enthaltend 1 bis 75 Gew.-%, vorzugsweise 5 bis 50 Gew.-%, bezogen auf die Lösung, eines erfindungsgemässen Polyarylenetherketons.

Die erfindungsgemässen Polyarylenetherketone können in der für Thermoplaste üblichen Weise eingesetzt werden und zu Formkörpern, Filmen oder Folien oder Beschichtungen verarbeitet werden. Gegenstand vorliegender Erfindung sind somit auch die aus den erfindungsgemässen Polyarylenetherketonen hergestellten Gegenstände, wie Formstoffe, Beschichtungen oder Folien.

Vor der Verarbeitung der beispielsweise als Schmelze oder insbesondere als Lösung vorliegenden Polyarylenetherketone können übliche Zusatzstoffe, wie beispielsweise Füllstoffe, Pigmente, Stabilisatoren oder Verstäkungsmittel, wie Kohlenstoff-, Bor-, Metall- oder Glasfasern, zugegeben werden.

Die erfindungsgemässen Polyarylenetherketone können auch als Matrixharze für die Herstellung von Faserverbundsystemen verwendet werden, wobei man als Verstärkungsfasern die üblichen bei der Verstärkung technischer Werkstoffe verwendeten Fasern einsetzen kann. Diese können organische oder anorganische Fasern, Naturfasern oder Synthesefasern sein, und als Faserbündel, als orientierte oder nicht-orientierte Fasern oder als Endlosfasern vorliegen.

Eine weitere bevorzugte Anwendungsmöglichkeit für die erfindungsgemässen Polyarylenetherketone besteht aufgrund ihrer Löslichkeit in der Modifizierung anderer Matrixharze. Diese können Thermoplaste oder Duromere sein. So wird beispielsweise bei der Modifizierung von duromeren Matrixharzen mit thermoplastischen Polymeren im allgemeinen mit möglichst konzentrierten Lösungen dieser Polymeren in herkömmlichen organischen Lösungsmitteln gearbeitet. Die Lösungsmittel sollen nach dem Einarbeiten rasch entfernbar sein und deshalb möglichst niedrige Siedepunkte und hohe Flüchtigkeiten aufweisen.

Die Verwendung der erfindungsgemässen Polyarylenetherketone zum Modifizieren von thermoplastischen und duromeren Matrixharzen stellt einen weiteren Erfindungsgegenstand dar.

Herstellung der Monomeren

2-Jod-4′-fluorbenzophenon

Zu einer Suspension von 247 g (1,85 Mol) $AlCl_3$ und einer katalytischen Menge $FeCl_3$ (1,85 g) in 420 ml Fluorbenzol werden bei Raumtemperatur 493 g (1,85 Mol) 2-Jodbenzoylchlorid gelöst in 420 ml Fluorbenzol zugetropft. Unter Stickstoff wird 20 Stunden bei Raumtemperatur gerührt. Ueberschüssiges Fluorbenzol wird aus dem Reaktionskolben abdestilliert und der Rückstand unter Rühren auf 5 Liter Wasser gegossen. Der Niederschlag wird abfiltriert, mit Wasser gewaschen und im Vakuumtrockenschrank bei 40°C getrocknet. Eine Umkristallisation aus Hexan ergibt 501 g (83 % der Theorie) 2-Jod-4′-fluorbenzophenon vom Schmelzpunkt 51-52°C.

Elementaranalyse berechnet für $C_{13}H_8FJO$:

| | | | |
|---|---|---|---|
| Ber.: | C = 47,88 % | Gef.: | C = 47,72 % |
| | H = 2,47 % | | H = 2,77 % |
| | J = 38,91 % | | J = 39,00 %. |

2,2'-Bis-(4-fluorbenzoyl)-biphenyl

Eine Mischung von 114,3 g (0,35 Mol) 2-Jod-4'-fluorbenzophenon und 343 g feines Kupferpulver wird 30 Minuten auf 200°C erhitzt. Nach Abkühlung auf 100°C werden 300 ml Toluol zugegeben und die Lösung wird heiss filtriert. Der Rückstand wird gut mit heissem Toluol gewaschen und die vereinigte Toluolphase im Rotationsverdampfer eingedampft. Umkristallisation des Rückstandes aus Ethanol ergibt 54 g (77 % der Theorie) 2,2'-Bis-(4-fluorbenzoyl)-biphenyl vom Schmelzpunkt 141-142°C.

Elementaranalyse berechnet für $C_{26}H_{16}F_2O_2$:

| | | | |
|---|---|---|---|
| Ber.: | C = 78,38 % | Gef.: | C = 78,31 % |
| | H = 4,05 % | | H = 4,18 % |

2-Jod-4'-methoxy-benzophenon

Eine Suspension von 493 g (1,85 Mol) 2-Jodbenzoylchlorid und einer katalytischen Menge $FeCl_3$ (6 g) in 1000 ml Anisol wird während 3 Stunden am Rückfluss gekocht. Nach Abkühlen auf Raumtemperatur wird die Reaktionslösung mit 1 Liter Toluol verdünnt und mit 2 Liter Wasser extrahiert. Die organische Phase wird über $Na_2SO_4$ getrocknet und im Rotationsverdampfer eingedampft. Der Rückstand wird unter Rühren auf 2 Liter Isopropanol gegossen, der Niederschlag abfiltriert und im Vakuumschrank bei 50°C getrocknet. Man erhält 461 g (74 % der Theorie) 2-Jod-4'-methoxy-benzophenon vom Schmelzpunkt 84-85°C.

Elementaranalyse berechnet für $C_{14}H_{11}IO_2$:

| | | | |
|---|---|---|---|
| Ber.: | C = 49,73 % | Gef.: | C = 49,95 % |
| | H = 3,28 % | | H = 3,28 % |
| | J = 37,53 % | | J = 37,58 %. |

2,2'-Bis-(4-methoxybenzoyl)-biphenyl

Eine Mischung von 181,2 g (0,535 Mol) 2-Jod-4'-methoxybenzophenon und 362 g feines Kupferpulver wird 1 Stunde auf 200°C erhitzt. Nach Abkühlen auf 100°C werden 700 ml Toluol zugegeben und die Lösung wird heiss filtriert. Der Rückstand wird gut mit heissem Toluol gewaschen und die vereinigte Toluolphase im Rotationsverdampfer eingedampft. Umkristallisation des Rückstandes aus Ethanol ergibt 90 g (80 % der Theorie) 2,2'-Bis-(4-methoxybenzoyl)-biphenyl vom Schmelzpunkt 156-158°C.

Elementaranalyse berechnet für $C_{28}H_{22}O_4$:

| | | | |
|---|---|---|---|
| Ber.: | C = 79,60 % | Gef.: | C = 79,62 % |
| | H = 5,25 % | | H = 5,37 %. |

2,2'-Bis-(4-hydroxybenzoyl)-biphenyl

Zu einer Lösung von 89 g (0,21 Mol) 2,2'-Bis-(4-methoxybenzoyl)-biphenyl in 1,4 Liter Toluol werden bei Raumtemperatur 154 g (1,15 Mol) $AlCl_3$ portionenweise zugegeben. Die Suspension wird 16 Stunden bei 80°C unter Stickstoff gerührt. Nach Abkühlen auf Raumtemperatur wird die Suspension unter Rühren auf 2 Liter Wasser gegossen. Der sich bildende Niederschlag wird abfiltriert und zuerst gut mit Wasser und dann mit Toluol gewaschen. Nach Trocknen im Vakuumtrockenschrank bei 80°C verbleiben 78 g (94 % der Theorie) 2,2′-Bis-(4-hydroxybenzoyl)-biphenyl vom Schmelzpunkt 259-261°C.

Elementaranalyse berechnet für $C_{26}H_{18}O_4$:

| | | | |
|---|---|---|---|
| Ber.: | C = 79,17 % | Gef.: | C = 79,20 % |
| | H = 4,60 % | | H = 4,99 %. |

Beispiel 1: Polyarylenetherketonsulfon aus 2,2′-Bis-(4-fluorbenzoyl)-biphenyl und 4,4′-Dihydroxydiphenylsulfon

In einem Rundkolben mit Rührer und Schutzgasanschluss wird unter Stickstoff eine Mischung aus 25,08 g (0,1002 Mol) 4,4′-Dihydroxydiphenylsulfon, 97,60 g Diphenylsulfon, 14,60 g (0,1056 Mol) Kaliumcarbonat und 51 g Xylol bei einer Badtemperatur von 200°C erhitzt und ein Xylol/Wasser-Gemisch abdestilliert. Gegen Ende des Destillationsvorganges wird dabei kurzzeitig Vakuum (2 mbar) angelegt. Sodann werden 39,84 g (0,1000 Mol) 2,2′-Bis-(4-fluorbenzoyl)-biphenyl zu der Reaktionsmischung gegeben, die Temperatur innerhalb von 25 Minuten (min) auf 250°C erhöht und dort 1 Stunde (h) belassen. Danach wird die Temperatur 1 h auf 275°C und anschliessend weiter auf 300°C erhöht. Diese Temperatur wird 4 h beibehalten, wobei die Reaktionsmischung in zunehmendem Masse viskos wird.

Nach Abkühlen wird das Reaktionsgemisch dem Kolben entnommen, pulverisiert, mit verdünnter Essigsäure versetzt und zunächst mit Wasser und dann mit Aceton extrahiert. Anschliessend wird das Polymer in Methylenchlorid gelöst, eine geringe Menge unlösliches Material abfiltriert und in Isopropanol ausgefällt. Das so gereinigte Polymer wird danach im Vakuumtrockenschrank bis zu einer Temperatur von 240°C getrocknet. Ein auf diese Weise hergestelltes Polyarylenetherketonsulfon besitzt eine reduziert Viskosität (1 % Polymeres in N-Methylpyrrolidon (NMP) bei 25°C, d.h., 1 g Polymeres in 100 ml NMP) von 0,67 dl/g. Es ist zu 20 Gew.-% in Methylenchlorid löslich. Die durch Differential Scanning Calorimetry (DSC) bestimmte Glasübergangstemperatur liegt bei 208°C.

Beispiel 2: Polyarylenetherketonsulfon-Copolymer aus 2,2′-Bis-(4-fluorbenzoyl)-biphenyl, 4,4′-Dichlordiphenylsulfon und 4,4′-Dihydroxydiphenylsulfon

In analoger Weise wie in Beispiel 1 wird ein Polyetherketonsulfon aus 4,4′-Dihydroxydiphenylsulfon (0,1001 Mol), 2,2-Bis-(4-fluorbenzoyl)-biphenyl (0,0250 Mol) und 4,4′-Dichlordiphenylsulfon (0,0750 Mol) mit Kaliumcarbonat (0,1061 Mol) hergestellt. Reaktionsbedingungen: 1 h/250°C, 1 h/275°C, 3 h/280°C. Das resultierende Polymere (red. Viskosität = 0,33 dl/g) besitzt eine Glasübergangstemperatur von 218°C. Es ist zu mehr als 25 Gew.-% in Methylenchlorid löslich.

Beispiel 3: Polyarylenetherketonsulfon-Copolymer aus 2,2′-Bis-(4-fluorbenzoyl)-biphenyl, 4,4′-Dichlordiphenylsulfon und 4,4′-Dihydroxydiphenylsulfon

In analoger Weise wie in Beispiel 1 wird ein Polyetherketonsulfon aus 4,4′-Dyhydroxydiphenylsulfon (0,4015 Mol), 2,2′-Bis-(4-fluorbenzoyl)-biphenyl (0,040 Mol) und 4,4′-Dichlordiphenylsulfon (0,360 Mol) mit Kaliumcarbonat (0,420 Mol) hergestellt. Reaktionsbedingungen: 1 h/250°C, 1 h/275°C und 5,5 h/280°C. Das resultierende Polymere (red. Viskosität = 0,87 dl/g) besitzt eine Glasübergangstemperatur von 227°C. Es ist zu mehr als 25 % in Methylenchlorid löslich.

Beispiel 4: Polyarylenetherketonsulfon aus 2,2′-Bis-(4-hydroxybenzoyl)-biphenyl und 4,4′-Dichlordiphenylsulfon

In analoger Weise wie in Beispiel 1 wird ein Polyetherketonsulfon aus 2,2′-Bis-(4-hydroxybenzoyl)-biphenyl (0,1003 Mol) und 4,4′-Dichlordiphenylsulfon (0,1002 Mol) mit Kaliumcarbonat (0,1085 Mol) hergestellt. Reaktionsbedinungen: 1 h/250°C, 1 h/273°C und 4 h/300°C. Das resultierende Polymere (red. Viskosität = 0,17 dl/g) besitzt eine Glasübergangstemperatur von 180°C. Es ist zu mehr als 25 % in Methylenchlorid löslich.

Beispiel 5: Polyarylenetherketonsulfon-Copolymer aus 2,2'-Bis-(4-hydroxybenzoyl)-biphenyl, 4,4'-Dihydroxydiphenylsulfon und 4,4'-Dichlordiphenylsulfon

In analoger Weise wie in Beispiel 1 wird ein Polyetherketonsulfon aus 2,2'-Bis-(4-hydroxybenzoyl)-biphenyl (0,0255 Mol), 4,4'-Dihydroxydiphenylsulfon (0,0754 Mol) und 4,4'-Dichlordiphenylsulfon (0,1001 Mol) mit Kaliumcarbonat (0,1051 Mol) hergestellt. Reaktionsbedingungen: 1 h/252°C, 1 h/278°C und 4 h/302°C. Das resultierende Polymere (red. Viskosität = 0,33 dl/g) besitzt eine Glasübergangstemperatur von 215°C. Es ist zu mehr als 25 % in Methylenchlorid löslich.

Beispiel 6: Polyarylenetherketonsulfon-Copolymer aus 2,2'-Bis-(4-hydroxybenzoyl)-biphenyl, 4,4'-Dihydroxydiphenylsulfon und 4,4'-Dichlordiphenylsulfon

In analoger Weise wie in Beispiel 1 wird ein Polyetherketonsulfon aus 2,2'-Bis-(4-hydroxybenzoyl)-biphenyl (0,0400 Mol), 4,4'-Dihydroxydiphenylsulfon (0,3615 Mol) und 4,4'-Dichlordiphenylsulfon (0,400 Mol) mit Kaliumcarbonat (0,4200 Mol) hergestellt. Reaktionsbedingungen: 1 h/250°C, 1 h/275°C und 4 h 20 min/280°C. Das resultierende Polymere (red. Viskosität = 0,60 dl/g) besitzt eine Glasübergangstemperatur von 226°C. Es ist zu mehr als 25 % in Methylenchlorid löslich.

Beispiele 7-14:

Die in nachfolgender Tabelle zusammengestellten Beispiele werden in analoger Weise wie in Beispiel 1 durchgeführt.

Tabelle 1: Zusammensetzung und Eigenschaften von Polyaryleneetherketonsulfonen

| Bsp. Nr. | Zusammensetzung | Reaktions- bedingungen | red. Visk. [dl/g] | Glasübergangs- temperatur (DSC) [°C] | Löslichkeit in $CH_2Cl_2$ [%] |
|---|---|---|---|---|---|
| 7 | 2,2'-Bis-(4-hydroxybenzoyl)-biphenyl (0,0402 Mol)<br>4,4'-Dihydroxydiphenylsulfon (0,3608 Mol)<br>2,2'-Bis-(4-fluorbenzoyl)-biphenyl (0,040 Mol)<br>4,4'-Dichlordiphenylsulfon (0,360 Mol)<br>Kaliumcarbonat (0,420 Mol) | 1 h/250°C<br>1 h/275°C<br>2 h 30 min/ 280°C | 0,95 | 225 | > 25 |
| 8 | 2,2'-Bis-(4-hydroxybenzoyl)-biphenyl (0,0501 Mol)<br>2,2'-Bis-(4-fluorbenzoyl-biphenyl (0,0501 Mol)<br>Kaliumcarbonat (0,0557 Mol) | 1 h/228°C<br>1 h/248°C<br>5 h/280°C | 0,23 | 185 | > 20 |

| Bsp. Nr. | Zusammensetzung | Reaktions-bedingungen | red. Visk. [dl/g] | Glasübergangs-temperatur (DSC) [°C] | Löslichkeit in $CH_2Cl_2$ [%] |
|---|---|---|---|---|---|
| 9 | 2,2'-Bis-(4-hydroxybenzoyl)-biphenyl (0,0502 Mol)<br>4,4'-Dihydroxybiphenyl (0,0502 Mol)<br>4,4'-Dichlordiphenylsulfon (0,1000 Mol)<br>Kaliumcarbonat (0,1100 Mol) | 1 h/250°C<br>1 h/275°C<br>2 h 15 min/ 280°C | 0,58 | 182 | > 25 |
| 10 | 4,4'Dihydroxydiphenylsulfon (0,1005 Mol)<br>2,2'-Bis-(4-fluorbenzoyl-biphenyl (0,0500 Mol)<br>4,4'-Difluorbenzophenon (0,0500 Mol)<br>Kaliumcarbonat (0,110 Mol) | 1 h/250°C<br>1 h/275°C<br>5 h 30 min/ 280°C | 0,29 | 191 | > 25 |

| Bsp. Nr. | Zusammensetzung | Reaktions-bedingungen | red. Visk. [dl/g] | Glasübergangs-temperatur (DSC) [°C] | Löslichkeit in $CH_2Cl_2$ [%] |
|---|---|---|---|---|---|
| 11 | 9,9-Bis-(4-hydroxyphenyl)-fluoren (0,0501 Mol)<br><br>2,2'-Bis-(4-fluorbenzoyl-biphenyl (0,0500 Mol)<br><br>Kaliumcarbonat (0,0525 Mol) | 1 h/250°C<br><br>1 h/275°C<br><br>3 h 280°C | 0,42 | 221 | > 25 |
| 12 | 6,6'-Dihydroxy-3,3,3',3'-tetramethyl-1,1'-spirobiindan (0,0501 Mol)<br><br>2,2'-Bis-(4-fluorbenzoyl)-biphenyl (0,0500 Mol)<br><br>Kaliumcarbonat (0,0525 Mol) | 1 h/250°C<br><br>1 h/275°C<br><br>4 h 30 min/ 280°C | 0,21 | 199 | > 25 |

| Bsp. Nr. | Zusammensetzung | Reaktions-bedingungen | red. Visk. [dl/g] | Glasübergangs-temperatur (DSC) [°C] | Löslichkeit in $CH_2Cl_2$ [%] |
|---|---|---|---|---|---|
| 13 | 4,4'-Dihydroxydiphenylsulfon (0,1004 Mol)<br>2,2'-Bis-(4-fluorbenzoyl-biphenyl (0,0500 Mol)<br>2,6-Dichlorbenzonitril (0,0500 Mol)<br>Kaliumcarbonat (0,110 Mol) | 1 h/250°C<br>1 h/275°C<br>4 h 280°C | 0,30 | 205 | > 25 |
| 14 | 2,2'-Bis-(4-hydroxybenzoyl)-biphenyl (0,0201 Mol)<br>4,4'-Dihydroxydiphenylsulfon (0,1811 Mol)<br>2,6-Difluorbenzonitril (0,0201 Mol)<br>4,4'-Dichlordiphenylsulfon (0,1800 Mol)<br>Kaliumcarbonat (0,2205 Mol) | 1 h/228°C<br>1 h/250°C<br>3 h/283°C | 0,98 | 231 | > 25 |

Beispiel 15:

Das nach Beispiel 3 synthetisierte Polymere wird als Lösung in Methylenchlorid, enthaltend 20 bzw. 30 Gewichtsteile Polymeres, zu einer Mischung, bestehend aus 50 Gewichtsteilen Tetraglycidyldiaminodiphenylmethan und 50 Gewichtsteilen Triglycidyl-p-aminophenol, gegeben und das Lösungsmittel im Vakuum entfernt. Nach Zugabe von 50 Gewichtsteilen p-Diaminodiphenylsulfon wird die Mischung 2 Stunden bei 160°C sowie 2 Stunden bei 210°C ausgehärtet. Aus einer so hergestellten Platte werden Prüfkörper geschnitten und die Bruchzähigkeit als "bend notch" gemäss ASTM E 399 bestimmt.

Es ergeben sich die folgenden Werte:

| Zusatz Thermoplast (Gewichtsteile) | Bruchzähigkeit ($J/m^2$) |
|---|---|
| 20 | 273 |
| 30 | 402 |

**Patentansprüche**

1. In halogenierten Kohlenwasserstoffen lösliche Polyarylenetherketone mit einer reduzierten Viskosität von 0,1 bis 2,0 dl/g, gemessen bei 25°C an einer 1-%igen Lösung in N-Methylpyrrolidon (NMP), die, bezogen auf die Gesamtmenge der im Polyarylenetherketonharz vorhandenen Strukturelemente, 1-100 Mol-% eines wiederkehrenden Strukturelementes der Formel I oder II

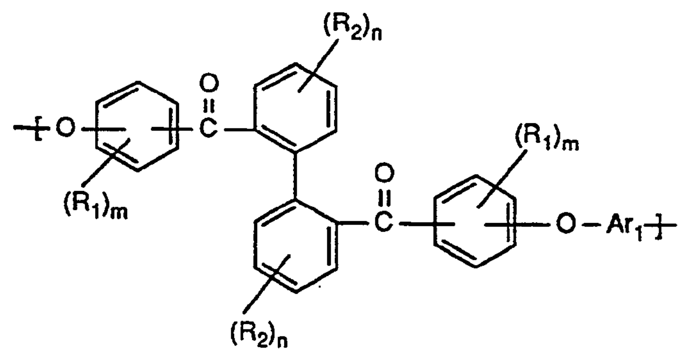

(I),

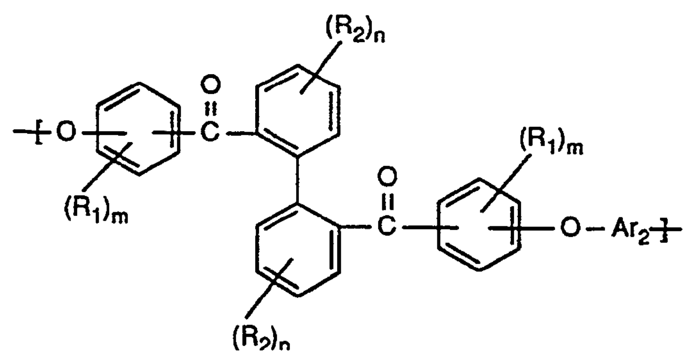

(II)

und 99-0 Mol-% eines wiederkehrenden Strukturelementes der Formel III

$$\{O-Ar_2-O-Ar_1\} \qquad (III)$$

enthalten, worin $R_1$ ein $C_1$-$C_4$-Alkyl, $C_1$-$C_4$-Alkoxy, $C_6$-$C_{12}$-Aryl, $C_2$-$C_4$-Alkenyl oder Halogenatom bedeutet, wobei m für Null oder eine Zahl von 1-4 steht, $R_2$ ein $C_1$-$C_4$-Alkyl, $C_1$-$C_4$-Alkoxy, $C_6$-$C_{12}$-Aryl oder Haloge-

natom bedeutet, wobei n für Null oder eine Zahl von 1-4 steht, $Ar_1$ ein zweiwertiger Rest einer diphenolischen Verbindung und $Ar_2$ ein zweiwertiger Rest einer aktivierten, zur nucleophilen Austauschreaktion befähigten Dihalogenverbindung sind.

2. Polyarylenetherketone gemäss Anspruch 1, worin $Ar_1$ in den Strukturelementen der Formeln I und III eine unsubstituierte oder durch ein oder mehrere $C_1$-$C_4$-Alkyle, $C_1$-$C_4$-Alkoxy oder Halogenatome substituierte Gruppe der Formeln IVa bis IVg

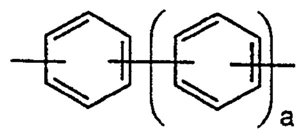 (IVa),

wobei a Null oder die Zahl 1 ist,

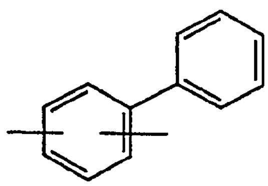 (IVb), 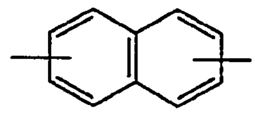 (IVc),

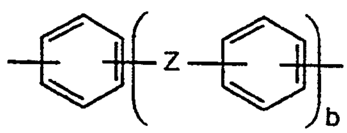 (IVd),

wobei b die Zahl 1 oder 2 ist,

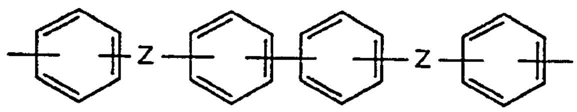 (IVe),

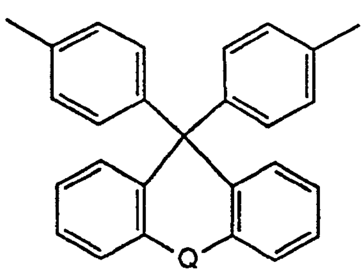 (IVf) oder

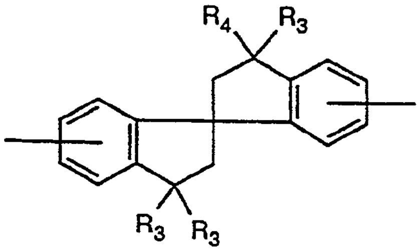 (IVg)

darstellt, worin Z für

$-CO-$, $-SO_2-$, $-SO-$, $-S-$, $-O-$, $-C(CH_3)_2$ , $-C(CF_3)_2$ , $-CH_2-$,

$-C(C_6H_5)-CH_3$ oder $-PO(R_4)$ ,

Q für eine direkte Bindung, -O-, $-CH_2-$ oder -CO-, $R_3$ je für ein $C_1$-$C_4$-Alkyl und $R_4$ für ein $C_1$-$C_4$-Alkyl oder Phenyl stehen.

3. Polyarylenetherketone gemäss Anspruch 1, worin $Ar_2$ in den Strukturelementen der Formeln II und III eine unsubstituierte oder durch ein oder mehrere $C_1$-$C_4$-Alkyle, $C_1$-$C_4$-Alkoxy oder Halogenatome substituierte Gruppe der Formel IVh bis IVl

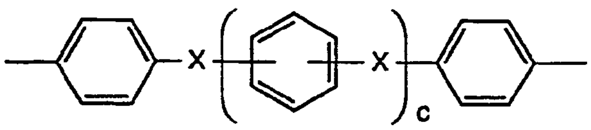

(IVh),

wobei c für Null oder die Zahl 1 oder steht,

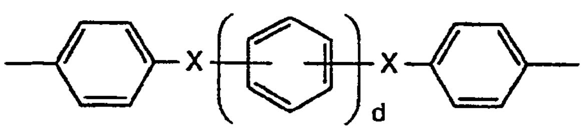

(IVi),

wobei d für die Zahl 2 oder 3 und X für -CO-, $-SO_2-$ oder -SO- stehen,

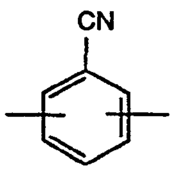

(IVj),

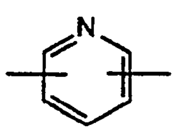

(IVk) oder

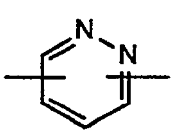

(IVl)

darstellt.

4. Polyarylenetherketone gemäss Anspruch 1, worin $R_1$ ein $C_1$-$C_4$-Alkyl, $C_1$-$C_4$-Alkoxy, $C_6$-$C_{12}$-Aryl, $C_2$-$C_4$-Alkenyl oder Halogenatom bedeutet, wobei m für Null oder eine Zahl von 1-4 steht, $R_2$ ein $C_1$-$C_4$-Alkyl, $C_1$-$C_4$-Alkoxy, $C_6$-$C_{12}$-Aryl oder Halogenatom bedeutet, wobei n für Null oder eine Zahl von 1-4 steht, und $Ar_1$ eine unsubstituierte oder durch ein oder mehrere $C_1$-$C_4$-Alkyle, $C_1$-$C_4$-Alkoxy oder Halogenatome substituierte Gruppe der Formeln IVa bis IVe

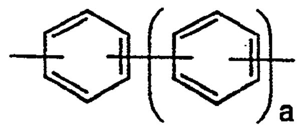

(IVa),

wobei a Null oder die Zahl 1 ist,

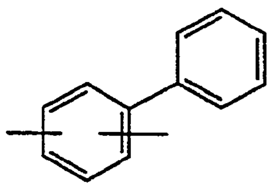

(IVb),

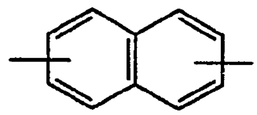

(IVc),

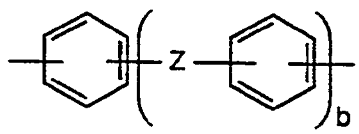

(IVd),

wobei b die Zahl 1 oder 2 ist, oder

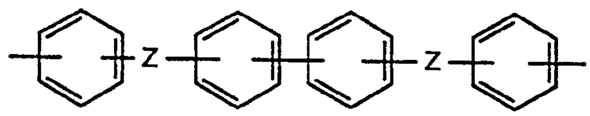

(IVe)

darstellt, worin Z für -CO-, $-SO_2-$, -SO-, -S-, -O-, $-C(CH_3)_2$ , $-C(CF_3)_2$ , $-CH_2-$ oder $-C(CH_3)(C_6H_5)-$ steht, und $Ar_2$ eine unsubstituierte oder durch ein oder mehrere $C_1$-$C_4$-Alkyle, $C_1$-$C_4$-Alkoxy oder Halogenatome substituierte Gruppe der Formel IVh oder IVi

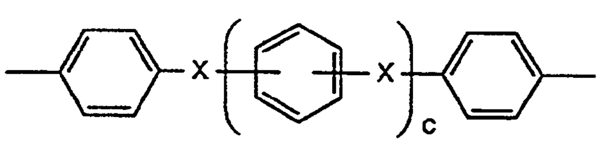

(IVh),

wobei c für Null oder die Zahl 1 oder steht,

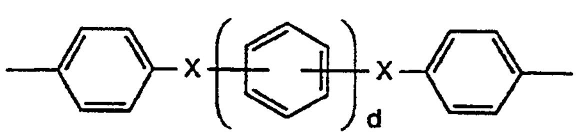

(IVi),

wobei d für die Zahl 2 oder 3 und X für -CO-, $-SO_2-$ oder -SO- stehen.

**5.** Polyarylenetherketone gemäss Anspruch 1, enthaltend 5-100 Mol-% eines wiederkehrenden Strukturelementes der Formel I oder II und 95-0 Mol-% eines wiederkehrenden Strukturelementes der Formel III.

6. Polyarylenetherketone gemäss Anspruch 1, worin $Ar_1$ in den Strukturelementen der Formeln I und III für einen Rest der Formeln

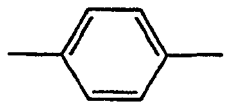 , 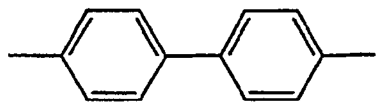 , 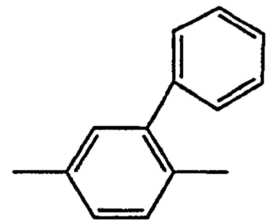 ,

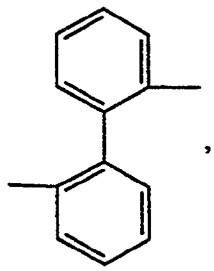 , 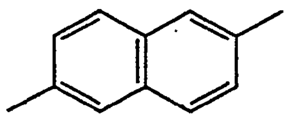 , 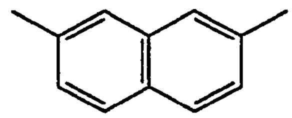 ,

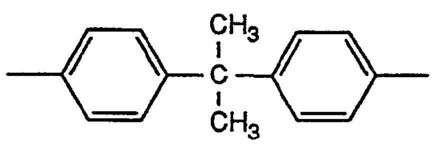 , 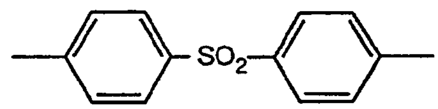 ,

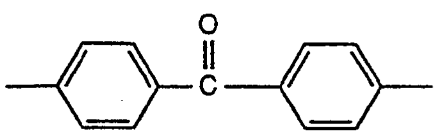 , 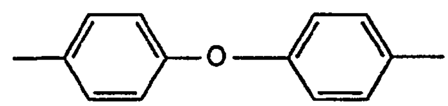 ,

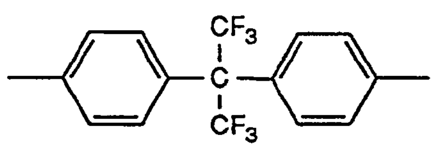 , 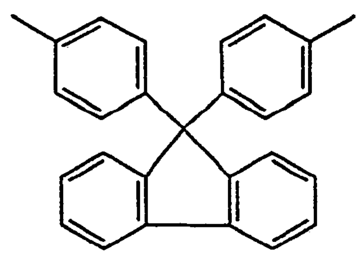 oder

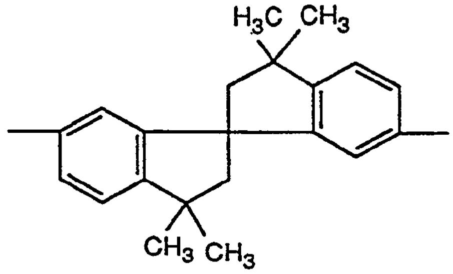 steht.

7. Polyarylenetherketone gemäss Anspruch 1, worin $Ar_1$ in den Strukturelementen der Formeln I und III für einen Rest der Formeln

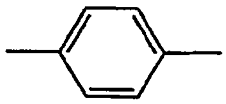 , 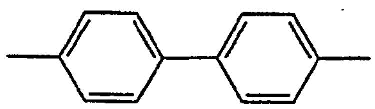 , 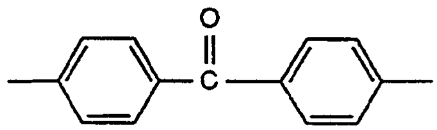 ,

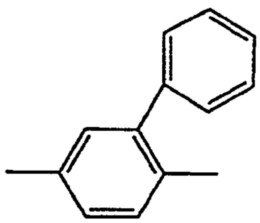 oder 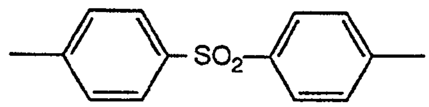 steht.

8. Polyarylenetherketone gemäss Anspruch 1, worin $Ar_2$ in den Strukturelementen der Formeln II und III für einen Rest der Formel

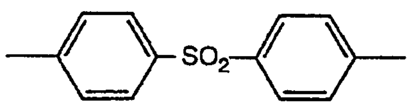 ,

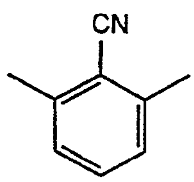 oder 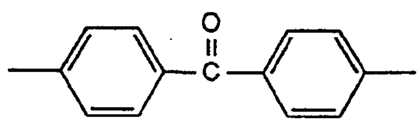 steht.

9. Polyarylenetherketone gemäss Anspruch 1, worin $Ar_1$ und $Ar_2$ in den Strukturelementen der Formeln I, II und III für den Rest der Formel

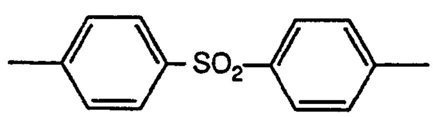

stehen.

10. Verfahren zur Herstellung von Polyarylenetherketonen gemäss Anspruch 1, dadurch gekennzeichnet, dass man

(a) eine Dihalogenverbindung der Formel V

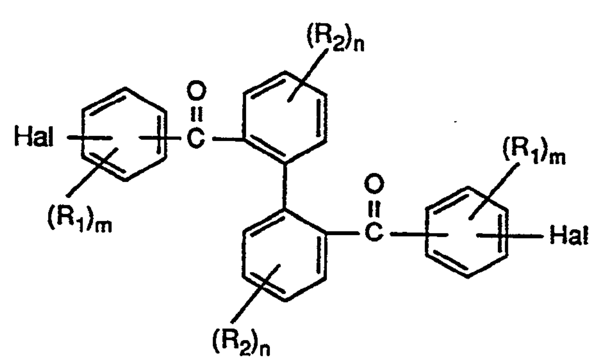 (V),

worin $R_1$, $R_2$, m und n die gleiche Bedeutung wie in Anspruch 1 haben und Hal für ein Halogen, insbesondere Fluor oder Chlor, steht, oder ein Gemisch aus einer Dihalogenverbindung der Formel V und einer darin bis zu 99 Mol-% enthaltenden Dihalogenverbindung der Formel VI

Hal-$Ar_2$-Hal (VI),

worin $Ar_2$ die gleiche Bedeutung wie in Anspruch 1 hat und Hal für ein Halogen, vorzugsweise Fluor oder Chlor, steht, in äquimolaren Mengen mit einem Diphenol der Formel VII oder dessen Alkali- oder Erdalkaliphenolat

HO-$Ar_1$-OH (VII),

worin $Ar_1$ die gleiche Bedeutung wie in Anspruch 1 hat, in Gegenwart von Alkali und in einem aprotischen Lösungsmittel polykondensiert oder

(b) eine Verbindung der Formel VIII oder deren Alkali- oder Erdalkaliphenolate

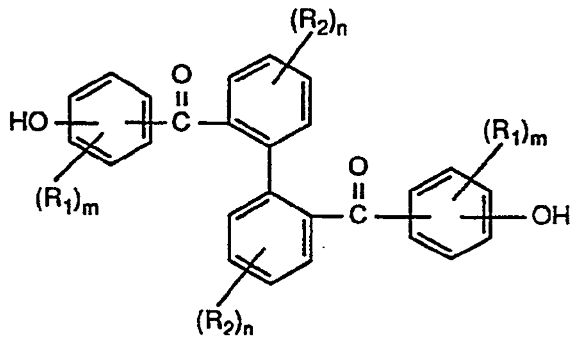

(VIII),

worin $R_1$, $R_2$, m und n die gleiche Bedeutung wie in Anspruch 1 haben, oder ein Gemisch aus einer Verbindung der Formel VIII und einem darin bis zu 99 Mol-% enthaltenen Diphenol der Formel VII in äquimolaren Mengen mit einer Dihalogenverbindung der Formel VI in Gegenwart von Alkali und in einem aprotischen Lösungsmittel polykondensiert, bis der erhaltene Polyarylenetherketon eine reduzierte Viskosität von 0,1 bis 2,0 dl/g aufweist, gemessen an einer 1-%igen Lösung in NMP bei 25°C.

**11.** Verfahren zur Herstellung von Verbindungen der Formeln V und VIII gemäss Anspruch 8, dadurch gekennzeichnet, dass man eine Verbindung der Formel IX

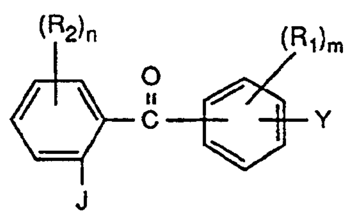

(IX),

worin $R_1$, $R_2$, m und n die gleiche Bedeutung wie in Formel V oder VIII haben und Y für ein Halogenatom oder $C_1$-$C_4$-Alkoxy, vorzugsweise Fluor, Chlor oder $C_1$-$C_2$-Alkoxy, insbesondere Fluor oder -$OCH_3$, steht, in Gegenwart einer 0,5 bis 3-fachen Gewichtsmenge Kupferpulver, bezogen auf die Gewichtsmenge der Verbindung der Formel IX, auf 150-250°C erhitzt, wobei eine Verbindung der Formel X

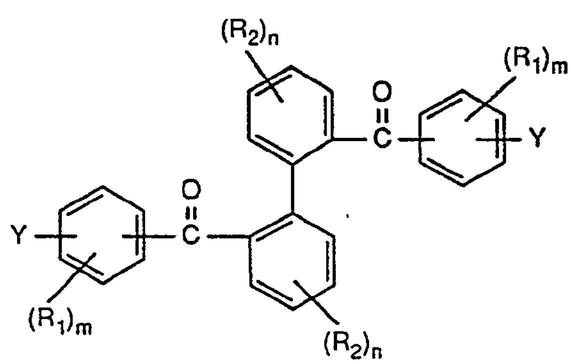

(X)

erhalten wird, worin $R_1$, $R_2$, m, n und Y die gleiche Bedeutung wie in Formel IX haben, und wenn Y für ein $C_1$-$C_4$-Alkoxy steht, diese Verbindung mit $AlCl_3$ in einem organischen Lösungsmittel unter Rühren bei etwa

60-160°C zu einer Verbindung der Formel VIII umsetzt, wobei pro 1 Mol der Verbindung der Formel X 3-6 Mole $AlCl_3$ eingesetzt werden.

**12.** Lösung enthaltend 1 bis 75 Gew.-%, bezogen auf die Lösung, eines Polymeren gemäss Anspruch 1, gelöst mit einem organischen Lösungsmittel.

**13.** Formstoffe, Beschichtungen oder Folien enthaltend ein Polyarylenetherketon gemäss Anspruch 1.

**14.** Verwendung der Polyarylenetherketone gemäss Anspruch 1 zum Modifizieren von thermoplastischen und duromeren Matrixharzen.

**Patentansprüche Für folgenden Vertragsstaat: ES**

**1.** Verfahren zur Herstellung von in halogenierten Kohlenwasserstoffen lösliche Polyarylenetherketone mit einer reduzierten Viskosität von 0,1 bis 2,0 dl/g, gemessen bei 25°C an einer 1-%igen Lösung in N-Methylpyrrolidon (NMP), die, bezogen auf die Gesamtmenge der im Polyarylenetherketonharz vorhandenen Strukturelemente, 1-100 Mol-% eines wiederkehrenden Strukturelementes der Formel I oder II

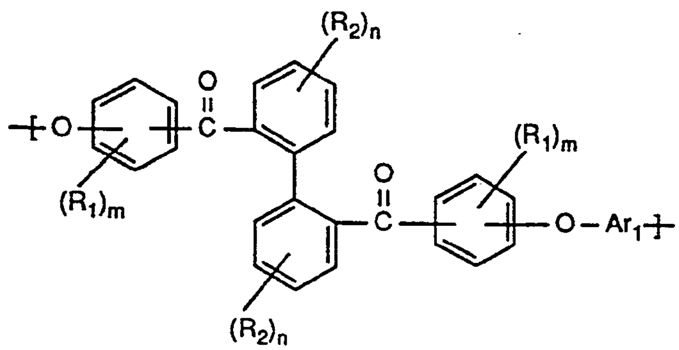

(I) oder

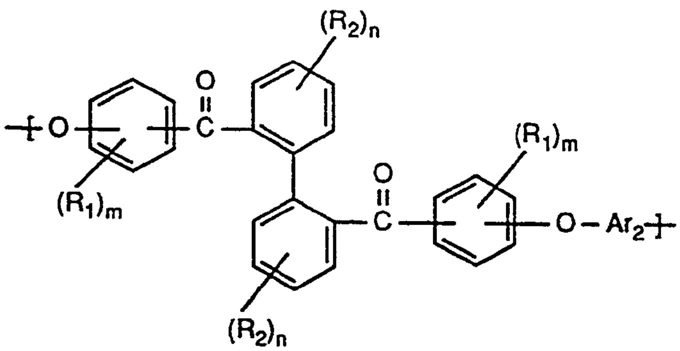

(II)

und 99-0 Mol-% eines wiederkehrenden Strukturelementes der Formel III

$$\left[ O\text{-}Ar_2\text{-}O\text{-}Ar_1 \right] \quad \text{(III)}$$

enthalten, worin $R_1$ ein $C_1$-$C_4$-Alkyl, $C_1$-$C_4$-Alkoxy, $C_6$-$C_{12}$-Aryl, $C_2$-$C_4$-Alkenyl oder Halogenatom bedeutet, wobei m für Null oder eine Zahl von 1-4 steht, $R_2$ ein $C_1$-$C_4$-Alkyl, $C_1$-$C_4$-Alkoxy, $C_6$-$C_{12}$-Aryl oder Halogenatom bedeutet, wobei n für Null oder eine Zahl von 1-4 steht, $Ar_1$ ein zweiwertiger Rest einer diphenolischen Verbindung und $Ar_2$ ein zweiwertiger Rest einer aktivierten, zur nucleophilen Austauschreaktion befähigten Dihalogenverbindung sind, dadurch gekennzeichnet, dass man

(a) eine Dihalogenverbindung der Formel V

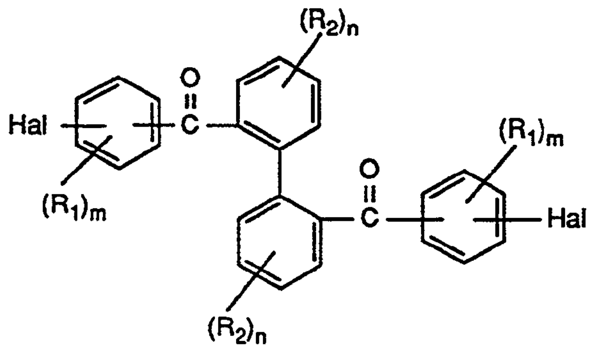

(V),

worin $R_1$, $R_2$, m und n die gleiche Bedeutung wie in Formel I oder II haben und Hal für ein Halogen, insbesondere Fluor oder Chlor, steht, oder ein Gemisch aus einer Dihalogenverbindung der Formel V und einer darin bis zu 99 Mol-% enthaltenden Dihalogenverbindung der Formel VI

$$Hal\text{-}Ar_2\text{-}Hal \qquad (VI),$$

worin $Ar_2$ die gleiche Bedeutung wie in Formel II hat und Hal für ein Halogen, vorzugsweise Fluor oder Chlor, steht, in äquimolaren Mengen mit einem Diphenol der Formel VII oder dessen Alkali- oder Erdakaliphenolat

$$HO\text{-}Ar_1\text{-}OH \qquad (VII),$$

worin $Ar_1$ die gleiche Bedeutung wie in Formel I hat, in Gegenwart von Alkali und in einem aprotischen Lösungsmittel polykondensiert oder

(b) eine Verbindung der Formel VIII oder deren Alkali- oder Erdalkaliphenolate

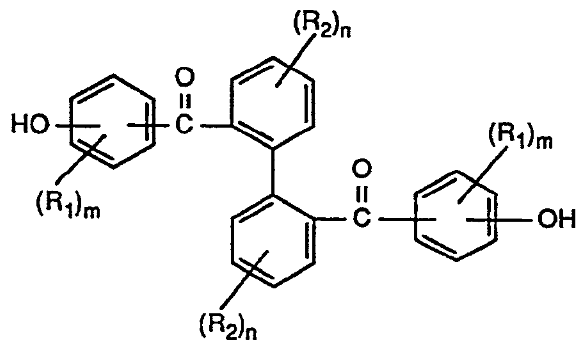

(VIII),

worin $R_1$, $R_2$, m und n die gleiche Bedeutung wie in Formel I oder II haben, oder ein Gemisch aus einer Verbindung der Formel VIII und einem darin bis zu 99 Mol-% enthaltenen Diphenol der Formel VII in äquimolaren Mengen mit einer Dihalogenverbindung der Formel VI in Gegenwart von Alkali und in einem aprotischen Lösungsmittel polykondensiert, bis der erhaltene Polyarylenetherketon eine reduzierte Viskosität von 0,1 bis 2,0 dl/g aufweist, gemessen an einer 1-%igen Lösung in NMP bei 25°C.

2. Verfahren gemäss Anspruch 1, dadurch gekennzeichnet, dass man

(a) eine Dihalogenverbindung der Formel V, worin $R_1$ ein $C_1$-$C_4$-Alkyl, $C_1$-$C_4$-Alkoxy, $C_6$-$C_{12}$-Aryl, $C_2$-$C_4$-Alkenyl oder Halogenatom bedeutet, wobei m für Null oder eine Zahl von 1-4 steht, $R_2$ ein $C_1$-$C_4$-Alkyl, $C_1$-$C_4$-Alkoxy, $C_6$-$C_{12}$-Aryl oder Halogenatom bedeutet, wobei n für Null oder eine Zahl von 1-4 steht, und Hal für ein Halogen, insbesondere Fluor oder Chlor, steht, oder ein Gemisch aus einer Dihalogenverbindung der Formel V und einer darin bis zu 99 Mol-% enthaltenden Dihalogenverbindung der Formel VI, worin $Ar_2$ eine unsubstituierte oder durch ein oder mehrere $C_1$-$C_4$-Alkyle, $C_1$-$C_4$-Alkoxy oder Halogenatome substituierte Gruppe der Formel IVh oder IVi

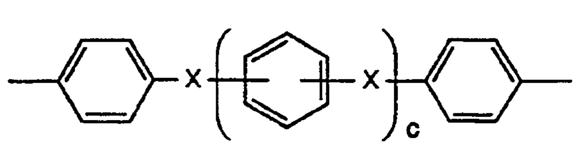

(IVh),

wobei c für Null oder die Zahl 1 oder steht,

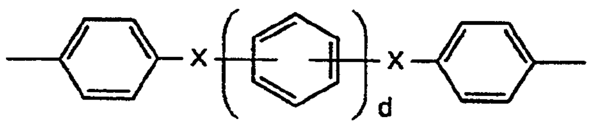

(IVi),

wobei d für die Zahl 2 oder 3 und X für -CO-, $-SO_2-$ oder -SO- stehen, und Hal für ein Halogen, vorzugsweise Fluor oder Chlor, steht mit einem Diphenol der Formel VII oder dessen Alkali oder Erdalkaliphenolate, worin $Ar_1$ eine unsubstituierte oder durch ein oder mehrere $C_1$-$C_4$-Alkyle, $C_1$-$C_4$-Alkoxy oder Halogenatome substituierte Gruppe der Formeln IVa bis IVe

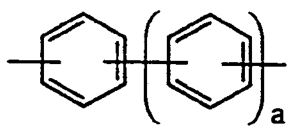

(IVa),

wobei a Null oder die Zahl 1 ist,

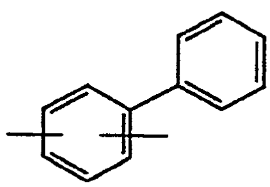

(IVb),

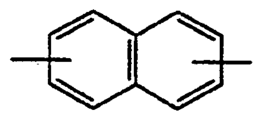

(IVc),

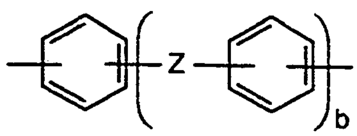

(IVd),

wobei b die Zahl 1 oder 2 ist, oder

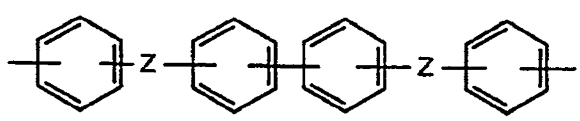

(IVe)

darstellt, worin Z für -CO-, $-SO_2-$, -SO-, -S-, -O-, $-\overset{|}{C}(CH_3)_2$ , $-\overset{|}{C}(CF_3)_2$ , $-CH_2-$ oder

$-\overset{|}{\underset{|}{C}}-CH_3$ steht, polykondensiert oder
$C_6H_5$

(b) eine Verbindung der Formel VIII oder deren Alkali- oder Erdalkaliphenolate, worin $R_1$, $R_2$, m und n die gleiche Bedeutung wie oben haben, oder ein Gemisch aus einer Verbindung der Formel VIII und einem darin bis zu 99 Mol-% enthaltenen Diphenol der Formel VII mit einer Dihalogenverbindung der Formel VI polykondensiert.